# EUROPEAN PATENT SPECIFICATION

(11) **EP 3 539 964 B1**
(45) Date of publication and mention of the grant of the patent: **10.11.2021**
(21) Application number: 16920991.3
(22) Date of filing: 27.12.2016
(51) Int. Cl.: C07D 473/34, C07D 473/18, C07D 239/54, C07D 239/47, A61K 9/127, A61K 48/00, C12N 15/88

(54) **BASE ACETAMIDE GLYCERIN ETHER MOLECULE, CHEMICAL SYNTHESIS METHOD THEREFOR, AND APPLICATIONS THEREOF IN FIELD OF GENE THERAPY**
BASE-ACETAMIDGLYCERINETHERMOLEKÜL, CHEMISCHES SYNTHESEVERFAHREN DAFÜR SOWIE ANWENDUNGEN DAVON AUF DEM GEBIET DER GENTHERAPIE
MOLÉCULE D'ÉTHER DE GLYCÉRINE D'ACÉTAMIDE DE BASE, SON PROCÉDÉ DE SYNTHÈSE CHIMIQUE ET SES APPLICATIONS DANS LE DOMAINE DE LA THÉRAPIE GÉNIQUE

(30) Priority: 09.11.2016 CN 201611002249
(43) Date of publication of application: 18.09.2019
(73) Proprietor: Beijing IntelliGene Biotech Limited Co., Haidian District Beijing 100094 (CN)
(72) Inventor: YANG, Zhenjun, Beijing 100191 (CN); WANG, Chao, Beijing 100191 (CN); SUN, jing, Beijing 100191 (CN)
(74) Representative: Ferreccio, Rinaldo
(86) International application number: PCT/CN2016/000712
(87) International publication number: WO 2018/085962

(56) References cited:
- CN-A- 102 925 487
- CN-A- 103 232 510
- CN-A- 103 242 243
- SKWARCZYNSKI, M. ET AL.: 'Thymine, adenine and lipoamino acid based gene delivery systems' CHEM. COMMUN vol. 46, 05 March 2010, pages 3140 - 3142, XP055503104

## Description

### Technical field

The invention relates to a base acetamide glycerin ether molecule and a chemical synthesis method thereof. The invention also relates to the application of such base acetamide glycerin ether molecules in the field of gene transfer. Such molecular structures have potential application prospects in the fields of biological materials and gene therapy. The invention belongs to the field of novel biological materials.

### Background technique

Compared with traditional protein-targeted small molecule drugs, nucleic acid drugs can regulate related physiological activities and behaviors at the genetic level, thereby fundamentally achieving the treatment and prevention of diseases. With the rapid development of research on nucleic acid drugs (such as antisense nucleic acids, siRNAs, nucleic acid aptamers, etc.), gene therapy associated with them has been favored in recent decades. Although nucleic acid drugs have great potential and application prospects, their obvious defects (poor stability, off-target effects, difficulty in transmembrane transport, etc.) hinder the further clinical application of nucleic acid drugs. Therefore, designing and constructing a system of nucleic acid drug delivery vehicles has become one of the focuses of current research.

Vectors that have been used for transmembrane transport of genes can be divided into two classes: viral vectors and non-viral vectors. Viral vectors are highly efficient in transfecting genes, but their preparations are difficult and may result in mutation or even canceration of the body cells, and toxic side effects are difficult to control. Most of the non-viral vectors are artificially synthesized. Although the transfection efficiency is relatively poor, but their variety is numerous and the structural performance is controllable, it is widely used in gene delivery. Non-viral vectors that have been used for gene delivery include cationic liposomes, cationic polymers, nanoparticles, and the like (Chem. Rev. 2009, 109, 259-302).

Cationic liposome is the most widely used non-viral gene carrier. It consists of an amphiphilic moiety. The cationic group acts as a polar head and the aliphatic long chain acts as a non-polar tail, relying on the Coulomb force between positive and negative charges. The combination of nucleic acids enables efficient encapsulation of nucleic acids. However, cationic liposomes have high cytotoxicity and serum toxicity due to the negative charge on the surface of the cell membrane and the large amount of electronegative proteins in the serum. In addition, due to the strong electrical effect, cationic liposomes bind tightly to nucleic acids and are difficult to release effectively after transmembrane transport *(*Biomaterials 2008, 29, 3477-3496). In order to avoid the defects of cationic liposome, it is very important to construct a novel carrier and use other forces instead of electrical effects to realize the binding of liposomes to nucleic acids.

In recent years, the use of hydrogen bonding to construct gene transfer vectors has received increasing attention. Milani et al. (J. Am. Chem. Soc. 2007, 129, 11664-11665) constructed a nucleotide phospholipid molecule using adenosine as a polar head and a phosphate bond at the 5'-hydroxyl position. The fatty acid glyceride binding via the phosphate bond acts as a non-polar tail. The nucleotide phospholipid molecule is capable of forming an ordered two-layered membrane-like structure in an aqueous solution, encapsulating the polyuracil nucleic acid strand by hydrogen bonding. Toth et al. (Chem. Commun. 2010, 46, 3140-3142) reported a novel molecule with a base as the head and a fatty chain as the tail, confirming its hydrogen bonding with the nucleic acid single strand. Chabaud et al. (Bioconjugate Chem. 2006, 17, 466-472) reported a class of cationic liposomes with nucleoside structures that have better gene transport effects. The authors believe that this effect is due to the hydrogen bonding between the base and the nucleic acid. Moreau et al. (J. Am. Chem. Soc. 2004, 126, 7533-7539) also reported a series of neutral nucleoside phospholipid molecules that form a gel structure in aqueous solution, having a effect of trapping DNA. No reports of transmembrane transport of liposome-encapsulated nucleic acids that rely on hydrogen bonding have been reported to date.

### Summary of the invention

One of the objects of the present invention is to provide a class of base acetamide glycerin ether molecules;

The second object of the present invention is to provide a chemical synthesis method for the above-mentioned base acetamide glycerin ether molecule;

The third object of the present invention is to provide the use of the above base acetamide glycerin ether molecule in the field of gene therapy.

The above objects of the present invention are achieved by the following technical solutions:
A base acetamide glycerin ether molecule having an amphiphilic structure represented by formula (I), that is, a base 1-acetic acid is used as a polar head and a 2, 3-dialkoxyl-1-propylamine is used as a non-polar tail, and the two parts are linked by an amide bond to form an amphiphilic structure which is both hydrophilic and lipophilic.

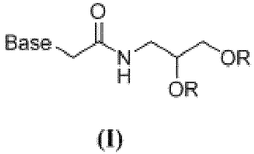

The base contained in the above base acetamide glycerin ether molecule, that is, the base group in the formula (I), is a common natural purine- or pyrimidine base, i.e, adenine, guanine, hypoxanthine, cytosine, thymine, pyrimidine and uracil.

The long chain aliphatic contained in the above base acetamide glycerin ether molecule, that is, the R group in the formula (I), is a saturated or unsaturated aliphatic carbon chain, having between 8 to 25 carbons in length.

A chemical synthesis method for preparing the above-mentioned base-acetamide glycerin ether molecule, wherein a base-1-acetic acid activated derivative represented by formula (II) and 2,3-dialkoxy-1-propylamine represented by formula (III) are raw materials, they react in an organic solvent to obtain a base acetamide glycerin ether molecule. A base-1-acetic acid activated derivative represented by formula (II), wherein the R' group is an activated structure such as chlorine or *N*-oxy-succinimide. In another material, 2, 3-dialkoxyl-1-propylamine shown in formula (III), the R groups are saturated or unsaturated aliphatic carbon chains having a length of between 8- and 25 carbons.

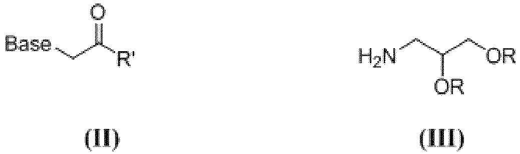

The above chemical synthesis method comprises the steps of: (i) commercially purchasing or preparing from simple materials the base-1-acetic acid activated derivative represented by the formula (II), and2, 3-dialkoxyl-1-propylamine represented by formula (III); (ii) carrying out the reaction between the base-1-acetic acid activating derivative as shown in formula (II), and 2,3-dialkoxy-1-propylamine as shown in formula (III) directly in an organic solvent, or by adding a base or an ester condensation catalyst to obtain a target product as shown in the formula (I).

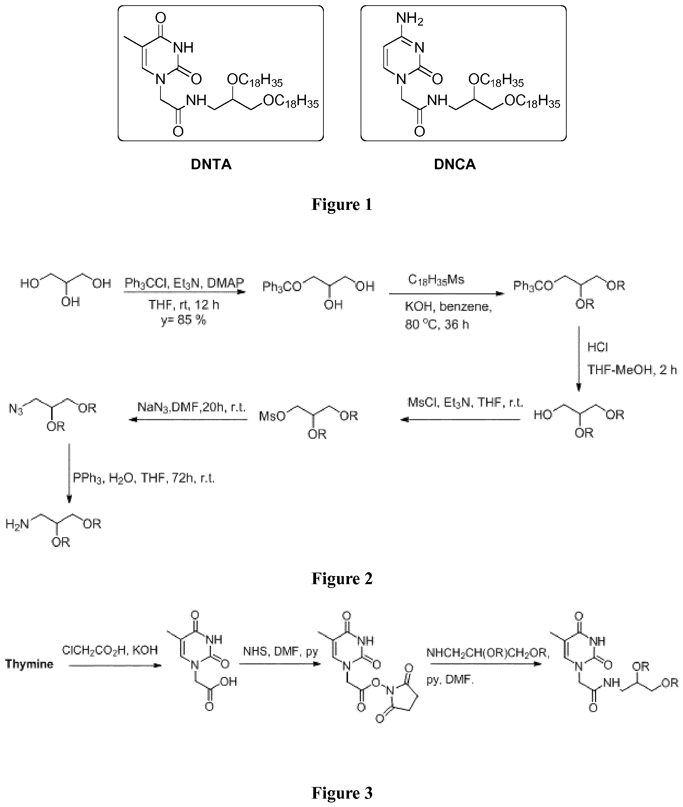

In the step (ii) of the above chemical synthesis method, preferably, the base-1-acetic acid-activated derivative represented by the formula (II) is gradually synthesized from a base; preferably, the carboxyl group is activated by N-hydroxysuccinimide. The 2,3-dialkoxy-1-propylamine represented by the formula (III) is gradually synthesized from glycerin and a long-chain aliphatic alcohol or a long-chain aliphatic alkyl bromide, and the synthesis route is shown in FIG 2.

In the above synthesis method, the solvent used in the step (ii) is acetonitrile, *N*,*N*-dimethylformamide, *N*-methylpyrrolidine, dichloromethane, dichloroethane, tetrahydrofuran, benzene, or other aprotic solvent; preferably, *N*,*N*-dimethylformamide is used as a solvent. Addition of 4-dimethylaminopyridine, triethylamine, pyridine, potassium hydroxide, or sodium hydroxide may be required for the reaction. Preferably, 4-dimethylaminopyridine is added as a catalyst, and pyridine or triethylamine is added as an organic base.

According to any one of the above description, the application of the base acetamide glycerin ether molecule in the preparation of a substance having a supramolecular structure, preferably, the supramolecular structure is a liposome.

Supramolecular structure is formed by the above-mentioned base acetamide glycerin ether molecules in an aqueous phase solvent. The molecules can be assembled into a supramolecular structure such as a liposome by a certain preparation method.

The above base acetamide glycerin ether molecules are used in the field of gene therapy, particularly in the preparation of transfection reagents for gene therapy. Experiments have shown that the compounds of the invention have no significant cytotoxicity. The compound contains a base in its head, which can bind to the nucleic acid by hydrogen bonding and electron cloud π-π stacking. These compounds have a hydrophilic and lipophilic amphiphilic structure, can be assembled into a supramolecular structure such as a liposome in an aqueous solution. They have a transmembrane ability, and can be used as a gene carrier to carry a nucleic acid drug into a cell membrane. Therefore, it can become a highly efficient biomaterial for agglomerating, trapping, bearing, carrying nucleic acid drugs, or mediating transmembrane transportation of nucleic acid drugs, thereby having broad application prospects in the field of gene therapy.

The present invention can achieve the following advantages: providing a class of base acetamide glycerin ether molecules, they are amphiphilic and capable of forming a liposome structure in an aqueous solution. The molecules have a base in their heads, which can bind nucleic acid through hydrogen bonding and electron cloud π-π stacking, and carries a nucleic acid across the membrane, so it is a highly efficient biological material with great potential. The raw materials used in the synthesis method provided by the invention are cheap and easy to obtain, and the synthesis method is simple and efficient. Such molecules have no obvious cytotoxicity, can form a liposome structure in an aqueous phase solvent, are simple to prepare. They have good capability to carry nucleic acids through cell membrane and have potential drug development prospects.

### Description of the figures:

Figure 1 Chemical structures of two base acetamide glycerin ether molecules;
Figure 2 Synthesis route of 2, 3-dialkoxyl-1-propylamine;
Figure 3 Synthesis route of the base acetamide glycerin ether molecule DNTA;
Figure 4 Synthesis route of the base acetamide glycerin ether molecule DNCA;
Figure 5 Scanning electron microscope images of DNTA (c, d) and DNCA (a, b) liposomes;
Figure 6 The growth inhibition rates of CCK8 cells caused by the base glycerol ether ester molecules DNCA and DNTA within 24 hour (left) and 72 hours (right);
Figure 7 Results of FAM-polyA and FAM-polyG transfection by DNTA and DNCA liposomes into cells;
Figure 8 Results of uptake of the antisense nucleic acid Cenersen by MCF-7 cells.

### Embodiments

The invention is further illustrated by the synthesis route of the compounds of the formula (I) according to the invention, and the various supramolecular structures formed thereof, in combination with specific embodiments, without limiting the scope of the invention.

### Example I Synthesis of base-acetamide glycerin ether molecules

### [Enbodiment 1] Synthesis of oleyl methylsulfonyl ester

Oleyl alcohol (50 g, purity 85%, 158 mmol), Et₃N (40 mL, 286 mmol) was added into a 1 L round bottom flask, subsequently DCM (500 mL) was added, stirred sufficiently on ice bath, causing temperature to drop to 0°C. Adding methanesulfonyl chloride (16 mL, 206 mmol) slowly thereto via a syringe, the solution became cloudy. Subsequently, remove the ice bath, and warm the reaction solution slowly to room temperature with continuous stirring for 12 h. The reaction was quenched by adding water (250 mL), and then separated the organic phase using a separation funnel. Back extract the aqueous phase with DCM (250 mL×2), pool the organic phases. Washed the pooled organic phases successively with IN aqueous hydrochloric acid (250 mL), 10% aqueous NaHCO₃ (250 mL) and brine (250 mL). The organic layer was evaporated to dryness under reduced pressure. The residue was separated with silica chromatographic column (the eluting agent: petroleum ether/ethyl acetate =20/1, R_{f}=0.3) to obtain pale yellow oily liquid 44.3g, yield: 81%. ¹H NMR (400 MHz, CDCl₃): δ 5.30-5.43 (m, 2H), 4.22 (t, J = 6.6 Hz, 2H), 3.00 (s, 3H), 1.90-2.10 (m, 4H) , 1.70-1.80 (m, 2H), 1.20-1.40 (m, 22H), 0.88 (t, J = 6.8 Hz, 3H); ¹³C NMR (100 MHz, CDCl₃): δ130.2, 129.9, 70.3 , 37.5, 32.0, 29.90, 29.83, 29.66, 29.46, 29.29, 29.26, 29.15, 27.36, 27.30, 25.6, 22.8, 14.3; IR (neat) 292 2925.5, 2854.5, 1463.6, 1355.9, 1175.4, 974.8, 947.8, 831.7, 721.6, 528.8; MS (ESI-TOF⁺) for C₁₉H₃₈O₃SNa [M+Na]⁺ found 369.2315, calcd 369.2434; Anal. calcd for C₁₉H₃₈O₃S: C 65.85, H 11.05, Found: C 65.63, H 10.98.

### [Embodiment 2] Synthesis of 1-triphenylmethoxyglycerol

Glycerin (40 g, 435 mmol), triphenylchloromethane (30 g, 107 mmol), DMAP (300 mg, 2.46 mmol) was placed in a dry 500 mL round bottom flask, THF (80 mL) and Et₃N (18 mL) were subsequently added, stirred at room temperature for 12 h. Water (100 mL) was added to the reaction mixture to quench the reaction, the mixture was then diluted with ethyl acetate (150 mL). After sufficient shaking, the mixture was transferred to a separating funnel, the organic phase was discarded. The aqueous phase was extracted with ethyl acetate (100 mL×2), the organic phase was pooled. The pooled organic phases were washed successively with saturated NaHCO₃ water solution (200 mL), water (200 mL) and saturated brine (200 mL). After filtration, the solvent was evaporated to dryness to obtain yellow oily material. The material was dissolved in toluene/n-hexane (200 mL, v/v = 1/1) and allowed to stand at room temperature for 24 h, crystallized into a white solid 29 g, yield: 85%. ¹H NMR (400 MHz, CDCl₃): δ 7.38-7.48 (m, 6H), 7.20-7.35 (m, 9H), 3.84 (s, ¹H), 3.63-3.71 (m, ¹H), 3.53- 3.63 (m, ¹H), 3.20-3.28 (m, 2H), 2.74 (brs, ¹H), 2.35 (brs, ¹H); ¹³C NMR (100 MHz, CDCl₃): δ 143.8, 128.7, 128.0, 127.3, 87.1, 71.3, 65.1, 64.4; IR (film, KBr) 338 3380.8, 3058.1, 2920.0, 2866.8, 1490.0, 1447.8, 1081.5, 1028.5, 699.8; MS (EI) for C₂₂H₂₂O₃ [M]⁺ found 334.5, calcd 334.2 ; Anal. calcd for C₂₂H₂₂O₃: C 79.02, H 6.63, Found: C 79.26, H 6.49.

### [Embodiment 3] Synthesis of 1-triphenylmethyl-2,3-dioleyl ether-glycerol

1-Triphenylmethoxy-glycerol-2,3-diol (8 g, 23.1 mmol), KOH (3.3 g, 58.9 mmol) and oleyl p-methylsulfonyl ester (19.2 g, 55.42 mmol) was mixed and dissolved in dry benzene (150 mL), added into a soxhlet extractor, heated to 80°C, and refluxed for 32 hours. 100 mL of ethyl acetate and 150 mL of water were subsequently added thereto, the organic phase was then separated. The aqueous layer was extracted with ethyl acetate (150 mL×3), the organic phases were pooled and desiccated with anhydrous Na₂SO₄. After the solvent evaporated to dryness, the residue was separated on a silica chromatographic column to obtain the target product 6.1 g, yield: 31%. Besides, 3.7 g 1-triphenylmethoxy-3-oleyl ether-glycerol-2-ol was obtained at a yield of 27%. The target product was a pale yellow liquid. ¹H NMR (400 MHz, CDCl₃): δ 7.40-7.50 (m, 6H), 7.18-7.32 (m, 9H), 5.26-5.43 (m, 4H), 3.50-3.60 (m, 5H), 3.35-3.45 (m, 2H), 3.12-3.20 (m, 2H), 1.92-2.08 (m, 8H), 1.50-1.58 (m, 4H), 1.26 (brs, 44H), 0.88 ( t, J = 6.6 Hz, 6H); ¹³C NMR (100 MHz, CDCl₃): δ 144.31, 130.07, 130.00, 128.90, 127.85, 127.02, 86.64, 78.45, 71.76, 71.33, 70.84, 63.73, 32.77, 32.06, 30.28 , 29.94, 29.93, 29.85, 29.82, 29.72, 29.68, 29.65, 29.47, 27.37, 27.06, 26.31, 26.25, 22.84, 14.27; IR (film, KBr) v 3004.4, 2925.3, 2854.1, 1742.6, 1597.7, 1490.7, 1450.0, 1220.6, 1118.7, 763.9, 745.0, 704.1, 632.8 cm⁻¹; MS (ESI-TOF⁺) for C₅₈H₉₀O₃Na [M+Na]⁺ found 857.9059, calcd 857.6782; Anal. calcd for C₅₈H₉₀O₃: C 83.39, H 10.86, Found: C 83.10, H 10.62.

### [Embodiment 4] Synthesis of 1,2-dioleyl ether-glycerol-3-ol

1-Triphenylmethyl-2,3-dioleyl ether-glycerol (8.34 g, 10 mmol) was suspended in a mixed solution of methanol-tetrahydrofuran (100 mL, v/v = 1/1), added with concentrated hydrochloric acid (2 mL, 12 M), stirred at room temperature for 2 h. After TLC test showed exhaustion of the starting material, the solvent was evaporated under reduced pressure. The residue was added with ethyl acetate (50 mL) and water (100 mL). The aqueous phase was extracted with EtOAc (3×100 mL), the organic phases were pooled and the pooled phase was desiccated with anhydrous Na₂SO₄. The desiccant was removed by filtration, and the solvent was evaporated to dryness. The residue was separated with silica chromatographic column(the eluting agent: petroleum ether/ethyl acetate =20/1, R_{f}=0.2) to obtain the target product 3.7g, yield: 62%, pale yellow oily liquid. ¹H NMR (400 MHz, CDCl₃): δ 5.30-5.45 (m, 4 H), 3.40-3.75 (m, 9 H), 2.18 (s, 1 H), 1.90-2.10 (m, 8 H), 1.55- 1.65 (m, 4 H), 1.25-1.40 (brs, 44 H), 0.88 (t, J = 6.4 Hz, 6 H); ¹³C NMR (100 MHz, CDCl₃): δ 130.10, 129.97, 78.39, 72.00, 71.07 70.54, 63.27, 32.06, 30.23, 29.92, 29.85, 29.81, 29.77, 29.67, 29.65, 29.60, 29.47, 29.41, 27.36, 26.25, 22.83, 14.25; IR (film, KBr) 347 3470.1, 3004.4, 2925.4, 2854.0, 1651.2, 1463.2, 1376.2, 1117.5, 1041.3, 968.0, 721.9 cm⁻¹; MS (ESI-TOF⁺) for C₃₉H₇₆O₃Na [M+Na]⁺ found 615.7213, calcd 615.5687; Anal. calcd for C₃₉H₇₆O₃: C 78.99, H 12.92, Found: C 78.72, H 12.68.

### [Embodiment 5] 1,2-dioleyl ether-3-glycerol mesylate

Triethylamine (0.54 mL, 3.91 mmol), 1,2-dioleyl ether-glycerol-3-ol (1.932 g, 3.26 mmol) were added into a 25 mL single-neck bottle, added with dry dichloromethane (5 ml) The mixture was dissolved under magnetic stirring, the reaction system was subsequently cooled to 0°C in an ice water bath. methanesulfonyl chloride (0.3 mL, 3.91 mmol) was added dropwise, the reaction was continued at 0°C for 2 hours. The reaction mixture was added into saturated NaHCO₃ solution (50 mL). The organic phase was discarded, the water phase was extracted with CH₂Cl₂ (2×50 mL), pooled the organic phases, the poled organic phase was desiccated with anhydrous Na₂SO₄ over night, the desiccating agent was removed by filtration, the solvent was subsequently evaporated under reduce pressure to dryness, the residue was separated using a silica chromatographic column (elution phase: petroleum ether/methanol= 120/1) to obtain 2.02 mg target product(yield: 92.6%). ¹H NMR (400 MHz, CDCl₃) δ 5.37 (s, 4H), 4.40 (d, J = 10.9 Hz, ¹H), 4.27 (d, J = 10.7, 5.8 Hz, ¹H), 3.54 (d, J = 29.7, 27.4, 20.6 Hz, 8H), 3.06 (s, 3H), 2.03 (d, J = 5.2 Hz, 7H), 1.58 (s, 4H), 1.29 (d, J = 10.6 Hz, 44H), 0.90 (t, J = 6.1 Hz, 6H). ¹³C NMR (101 MHz, CDCl₃) δ 127.93, 127.27, 77.34, 77.22, 77.02, 76.70, 76.38, 71.90, 70.83, 69.08, 37.40, 32.62, 31.92, 29.95, 29.78, 29.71, 29.59, 29.54, 29.52, 29.33, 29.28, 27.23, 26.08, 26.02, 22.70, 14.13. MS (EI) for C₄₀H₇₈O₅S [M+Na]⁺ found 693.36, calcd 693.55;

### [Embodiment 6] 1,2-dioleyl ether-glycerol-3-azide

The dried compound 1,2-dioleyl ether-glycerol-3-methanesulfonate (970 mg, 1.44 mmol) was added to a 25 mL single-necked flask, added with dry DMF (8 mL), dissolved with magnetic stirring. NaN₃ (188 mg, 2.88 mmol) was added under argon protection, the reaction mixture was heated to 70°C for 15 hours, and was subsequently cooled to room temperature, diluted with acetone (30 mL), the reaction liquid was filter using diatomaceous earth, the filtrate was evaporated to dryness under reduced pressure. The residue was separated using a silica chromatographic column (eluting phase: petroleum ether/ ethyl acetate =200/1) to obtain 345.5 mg colorless oily target product (yield: 56%). ¹H NMR (400 MHz, CDCl₃) δ 5.37 (s, 4H), 3.60~3.36 (m, 9H), 2.31 (s, ¹H), 2.07~1.95 (m, 8H), 1.59 (d, J = 8.3 Hz, 4H), 1.30 (d, J = 10.8 Hz, 44H), 0.90 (s, 6H). ¹³C NMR (101 MHz, CDCl₃) δ 129.89 (d, J = 10.6 Hz), 77.90 (s), 77.45∼76.95 (m), 76.95∼76.86 (m), 76.70 (s), 71.79 (s), 70.66 (s), 70.11 (s), 52.08 (s), 32.62 (s), 31.92 (s), 30.38∼29.54 (m ), 29.37 (t, J = 11.7 Hz), 27.22 (s), 26.07 (d, J = 7.5 Hz), 22.70 (s), 14.13 (s). MS (EI) for C₃₉H₇₅N₃O₂ [M+Na]⁺ found 640.49, calcd 640.58.

### [Embodiment 7] 1,2-dioleyl ether-glycerol-3-amine

1,2-dioleyl ether-glycerol-3-azide (309 mg, 0.5 mmol) was added into dry THF (10 ml), dissolving with magnetic stirring, the solution was cooled to 0°C, added with LiAlH₄ ( 95 mg, 2.5 mmol) under argon gas protection, after reacting for 45 min, the solution was warmed to room temperature, the reaction was continued for 2.5 h, subsequently quenched by the addition of saturated Na₂SO₄ (0.7 mL) solution. The mixture was filtered over celite, the filtrate evaporated to dryness under reduced pressure. The residue was subjected to silica gel column chromatography (elution phase: methylene chloride/methanol = 50/1) to obtain 233.5 mg light yellow oily target product (yield: 79%).¹H NMR (400 MHz, CDCl₃): δ = 5.30-5.45 (m, 4H), 3.40-3.75 (m, 9H), 2.18 (s, 1H), 1.90-2.10 (m, 8H), 1.55 -1.65 (m, 4H), 1.25-1.40 (brs, 44H), 0.88 (t, J = 6.4 Hz, 6H); ¹³C NMR (100 MHz, CDCl₃): δ = 130.10, 129.97, 78.39, 72.00, 71.07, 70.54, 63.27, 32.06, 30.23, 29.92, 29.85, 29.81, 29.77, 29.67, 29.65, 29.60, 29.47, 29.41, 27.36, 26.25, 22.83, 14.25; MS (ESI-TOF⁺) for C₃₉H₇₇NO₂, [MH]⁻found 590.75, calcd 591.60;

### [Embodiment 8] Synthesis of (thymine-1-yl)-acetic acid

Thymine (10.0 g, 79.3 mmol) was suspended in H₂O (150 mL), with aqueous KOH (50 mL, 3.6 M) solution added. After the mixture being stirred at room temperature for 10 min, the solution gradually became clear. Subsequently, chloroacetic acid (15.0 g, 159 mmol) was added thereto, and the reaction solution was heated to reflux for 90 min. After the reaction solution was cooled to room temperature, it was acidified to pH 3 with concentrated hydrochloric acid, subsequently stood at 4°C overnight to obtain a white crystalline precipitate. The white crystalline precipitate was obtained by filtration, and dried in vacuo to obtain 4.5 g target product (yield 31%). ¹H NMR (400 MHz, DMSO-d₆): δ 13.11 (s, 1H), 11.34 (s, 1H), 7.50 (s, 1H), 4.37 (s, 2H), 1.75 (s, 3H); ¹³C NMR (100 MHz, DMSO-d₆): δ 169.6, 164.4, 151.0, 141.8, 108.4, 48.4, 11.9; IR (film, KBr) 318 3180.2, 3076.2, 3027.0, 2962.3, 2835.8, 1737.7, 1708.4, 1664.8 , 1631.9, 1418.3, 1356.3, 1201.7, 1147.0, 829.8, 566.9 cm⁻¹; MS (EI): m/z (%): 184.1 (39) [M+], 95.9 (100); Anal. Calcd for C₇H₈N₂O₄: C 45.66, H 4.38, N 15.21, Found: C 45.59, H 4.40, N 15.25.

### [Embodiment 9] Synthesis of (thymidine-1-yl)-acetyl-(N-hydroxysuccinimide)-ester

To a dry 25 mL eggplant bottle, (thymidine-1-yl)-acetic acid (3 g, 16.3 mmol) and dry DMF (30 mL) were added and stirred to dissolve. Subsequently, *N*-hydroxysuccinimide (2.38 g, 21 mmol) and *N*,*N'*-dicyclohexylcarbodiimide (DCC, 3.36 g, 16.3 mmol) were added thereto. After stirring at room temperature overnight, a large amount of a white precipitate was reaped. The precipitate was removed by filtration, the filtrate was evaporated to dryness and the latter dissolved into DMF (5 mL). Anhydrous diethyl ether (30 mL) was added thereto to precipitate a white solid. The solid was obtained by filtration and dried in vacuo to yield 4.6 g target product (yield: 61%). White solid. ¹H MR (400 MHz, DMSO-d6): δ 11.52 (s, 1H), 7.63 (s, 1H), 4.96 (s, 2H), 2.83 (brs, 4H), 1.77 (s, 3H); ¹³C NMR (100 MHz, DMSO-d₆): δ 169.8, 165.0, 164.2, 150.7, 140.8, 109.3, 46.4, 25.5, 11.9; IR (film, KBr) v 3154.4, 3003.4, 2830.5, 1827.4, 1785.4, 1739.8, 1697.2, 1467.8, 1422.8, 1382.8, 1358.6, 1213.7, 1106.8, 1065.1, 793.9, 651.3 cm⁻¹; MS (ESI-TOF⁺) for C₁₁H₁₁N₃O₆Na [M+Na]⁺ found 304.0489, calcd 304.0540; Anal. Calcd for C₁₁H₁₁N₃O₆: C 46.98, H 3.94, N 14.94, Found: C 46.75, H 3.96, N 14.95.

### [Embodiment 10] Synthesis of 1,2,-bis(oleyl)-glycero-3-amine-(thymidine-1-yl)-acetyl ester (DNTA, structural formula shown in Figure 1)

The synthesis route of DNTA is shown in Figure 3.

(Thymine-1-yl)-acetyl-(N-hydroxysuccinimide)-ester (280 mg, 1.0 mmol), 1,2,-bis(oleyl)-glycerol-3-amine (709 mg, 1.2 mmol), DMPA (14.6 mg, 0.1 mmol), pyridine (0.4 mL) and dry DMF (20 mL) were mixed. Under argon protection, the mixture was stirred overnight. Ethyl acetate (200 mL) was added thereto, the mixture was diluted with EtOAc (200mL) and removed to a separating funnel. The organic phase was washed with dilute hydrochloric acid (0.1M), saturated NaHCO₃ water solution, water and saturated NaCl solution, successively. The organic phase was then desiccated with anhydrous sodium sulfate. The desiccant was then removed by filtration, the filtrate was evaporated to dryness under reduced pressure. The residue was subjected to silica column chromatography (elution phase: methane dichloride/methanol=50/1) to obtain 537.5 mg target product ( yield: 71%). Light yellow oily. ¹H NMR (400 MHz, CDCl₃) δ 8.12 (s, 1H), 7.09 (s, 1H), 6.43 (s, ¹H), 5.38 (d, J = 18.8 Hz, 4H), 4.31 (s, 2H), 3.58 (s, 1H), 3.52-3.42 (m, 6H), 2.05-1.94 (m, 10H), 1.60 (s, 4H), 1.29 (d, J = 11.7 Hz, 45H), 0.89 (d, J = 6.7 Hz, 6H). (ESI-MS) for C₄₆H₈₃N₃O₅ [MH]⁻ found 756.61, calcd. 757.23. ¹³C NMR (100 MHz, CDCl₃): δ = 167.54, 163.69, 150.61, 140.18, 130.13, 129.98, 111.38, 76.35, 72.03, 70.89, 69.97, 65.77, 48.60, 32.06, 29.93, 29.78, 29.67, 29.61, 29.48, 27.37, 26.19, 14.25, 12.45; (ESI-MS) for C₄₆H₈₃N₃O₅ [MH]⁻ found 756.61, calcd. 757.23.

### [Embodiment 11] Synthesis of (4-N-(diphenylmethoxycarbonyl)-cytosine)-1-acetic acid

Cytosine (10.3 g, 90 mmol, 1.0 eq) was dissolved in DMF (90 mL) added with potassium *t*-butoxide (11.6 g, 103.5 mmol, 1.15 eq), the reaction mixture was heated to 100°C for 2 hours, and was subsequently cooled to 10°C, 2-bromoacetic acid benzyl ester (16.05 mL, 101 mmol, 1.12 eq) was added dropwise thereto over 30 minutes. After the addition was completed, the reaction system was warmed to room temperature and the reaction was continued for 12 hours. Acetic acid (5.9 mL, 103.5 mmol, 1.2 eq) was added into the reaction system to quench the reaction. The reaction mixture was subject to rotate evaporation to dryness. The residue was resuspended into H₂O (100 mL) and stirred for 4 hr and subject to filtration, the filtrate was washed with H₂O (4×150 mL) and dried to obtain cytosine-1-benzylacetic acid (20.6 g, 82 mmol, 1.0 eq), the latter was dissolved in DMF (160 mL) added with *N*,*N'*-carbonyldiimidazole (21.25g, 131.25 mmol, 1.6 eq). After the TLC detection showed that the reaction was completed, methanol was added, stirring was continued for 1.5 hours, and diphenylmethanol (19.65 g, 106.5 mmol, 1.3 eq) was added. The reaction system was heated to 60°C, and then two portions of diphenylmethanol (1.825 g each, 2×9.9 mmol, 2×0.12 eq) were added at an interval of a hour, the reaction was continued for 6 hours. Heating was stopped for 12 hours and quenched by the addition of methanol (4.65 g, 115 mmol, 1.4 eq). The reaction mixture was dried with rotate evaporation to dryness, the residue was recrystallized successively with ethanol and methanol to obtain (4-*N*-(Diphenylmethoxycarbonyl)-cytosine)-1-acetate (29.35 g, 62.5 mmol, 1.0 eq). The product was added into acetonitrile: MeOH:H₂O:EtOH (2:2:1:1, 350mL) mixed solvent system, heated up the mixture to facilitate solution, then the mixture was cooled to 0°C, an aqueous solution (196.8 mL) of LiOH (25.5 g, 0.61 mol, 9.7 eq) was added. After TLC detection showed completion of the reaction, quenched the reaction by adding citric acid (58.5 g, 303.5, 4.9 eq) aqueous solution (290 mL). 22.1 g of [4-N-(diphenylmethoxycarbonyl)-cytosine]-1-acetic acid was obtained. ¹H NMR (400 MHz, D₆-DMSO, 25°C) δ 8.03-8.01 (d, J = 7.5, ¹H, C6), 7.46 (d, J = 7.5, 4H, Ph), 7.38 (t, J = 7.5 , 4H, Ph), 7.3 (d, J = 7.5, 2H, Ph), 6.96 (d, J = 7.5, ¹H, C5), 6.82 (s, ¹H, CH-(C₆H₅)2), 4.50 (s, 2H, N-CH₂-CO); ¹³C NMR (100 MHz, D₆-DMSO, 25 °C) δ 169.9, 163.5, 155.5, 152.8, 151.1, 140.8, 129.0, 128.3, 126.9, 94.2, 71.9, 51.3; (ESI) calculated for C₂₀H₁₇N₃O₅: (M+Na⁺): 402.1060, found: 402.1010.

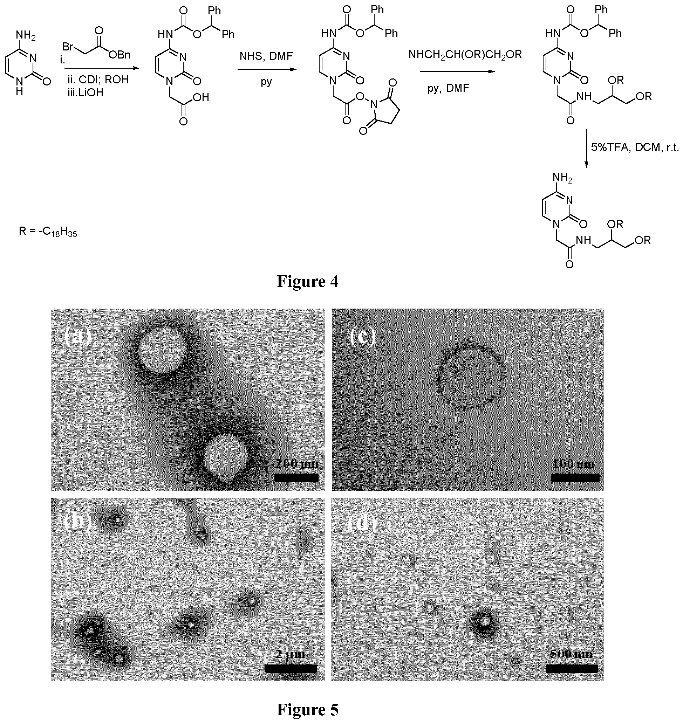

### [Embodiment 12] Synthesis of 1,2,-bis(oleyl)-glycero-3-amine-(cytosin-1-yl)-acetyl ester (DNCA, structural formula shown in Figure 1). Synthesis route of DNCA is shown in Figure 4.

(4-N-(Diphenylmethoxycarbonyl)-cytosine)-1-acetic acid (3.29 g, 10 mmol) and dry DMF (30 mL) were added to a dry 25 mL eggplant flask and stirred to complete dissolution. Then, *N*-hydroxysuccinimide (14.73 g, 13 mmol) and *N*,*N'*-dicyclohexylcarbodiimide (DCC, 2.06 g, 10 mmol) were added thereto. After stirring at room temperature overnight, a large amount of a white precipitate was found. The precipitate was removed by filtration, and then the filtrate was evaporated to dryness. Anhydrous diethyl ether (30 mL) was added thereto to precipitate a white solid. The solid was reaped by filtration and dried in vacuo to give 2.62 g target product (yield:55%). White solid. (4-*N*-(Diphenylmethoxycarbonyl)-cytosine)-(N-hydroxysuccinimide)-ester (476 mg, 1.0 mmol), 1,2,-di(oleyl)-glycerol 3-Amine (910 mg, 1.2 mmol), DMPA (14.6 mg, 0.1 mmol), pyridine (0.4 mL) and dry DMF (20 mL) were mixed. The mixture stirred at the room temperature under argon protection overnight, ethyl acetate (200 mL) was added to dilute the mixture, then the mixture removed to a separation funnel, washed successively with diluted hydrochloric acid (01.M), saturated NaHCO₃ water solution, water and saturated NaCl, solution. The organic phase was desiccated with anhydrous sodium sulfate, the desiccant was removed by filtration, then the filtrate was evaporated to dryness. The residue was separated with silica chromatographic column (elution phase: dichloromethane/methanol=50/1) to obtain 485.5 mg target product ( yield 51%). 1,2,-Di(Olefinyl)-glycero-3-amine-(4-*N*-(diphenylmethoxycarbonyl)-cytosine)-acetyl ester (476 mg, 0.5 mmol) was dissolved in CH₂Cl₂(20 mL) containing 5% TFA. The mixture was stirred at room temperature for 0.5 hour. The mixture was extracted with DCM (30 mL) and water (30 mL). The aqueous phase was re-extracted with DCM (2×30 mL), the organic phase was pooled. The pooled organic phase was washed successively with 10% NaHCO₃ (200 mL), water (200 mL) and brine (200 mL) and subsequently desiccated with anhydrous sodium sulfate overnight. The organic solvent was evaporated to dryness, the residue was separated with a silica chromatographic column (elution phase: CH₂Cl₂/methanol=50/1) to obtain the target product 345 mg (yield: 93%) , DNCA:¹H NMR (400 MHz, CDCl₃) δ 7.44-7.29 (m, 4H), 5.80 (d, J = 3.8 Hz, 1H), 5.37 (dd, J = 12.0, 7.4 Hz, 4H), 4.45 (s, 2H), 3.59-3.40 (m, 7H), 2.11-1.91 (m, 8H), 1.55 (s, 4H), 1.44-1.10 (m, 45H), 0.90 (t, J = 6.4 Hz, 6H). (ESI-MS ) for C₄₅H₈₂N₄O₄ [M+H]⁺ found 743.50, [M+Na]⁺ found 756.44. calcd. 742.21.

### Example II The preparation of liposome of base acetamide glycerin ether

The base-acetamide glycerin ether molecule has an amphiphilic structure and can be prepared into a supramolecular structure such as a liposome. Taking DNTA as an example, the preparation method of liposome is as follows: DNTA (1.18 mg, 1.56 µmol) is dissolved in methanol (1 mL), vortexed to fully dissolve, after centrifugation, DNTA methanol solution (12.8 µL) is measured out and adding to a 200 µL centrifuge tube, added with PBS (100 µL), vortexed (10 s) to mix well. The solution was placed in a PCR program for annealing. Procedure: Heating at 95°C for 10 minutes, cool down 5°C every 5 minutes until 15°C, then store at 4°C. After annealing was completed, the centrifuge tube was incubated at 40°C for 30 min for the complete evaporation of methanol to obtain the liposome solution.

Particle size and its morphology:
Under transmission electron microscope (TEM), it can be seen that the neutral base lipid carrier molecules DNCA and DNTA can form stable and uniform nanoparticles in aqueous solution. The size of the DNTA molecules assembled in water is around 150 nm. The size of the liposome formed by self-assembly of DNCA carrier molecules in water is around 180 nm (Fig. 5).

### Example III: Application of base-acetamide glycerin ether molecule in the field of gene therapy.

### 1. Cytotoxicity of base-acetamide glycerin ether molecules

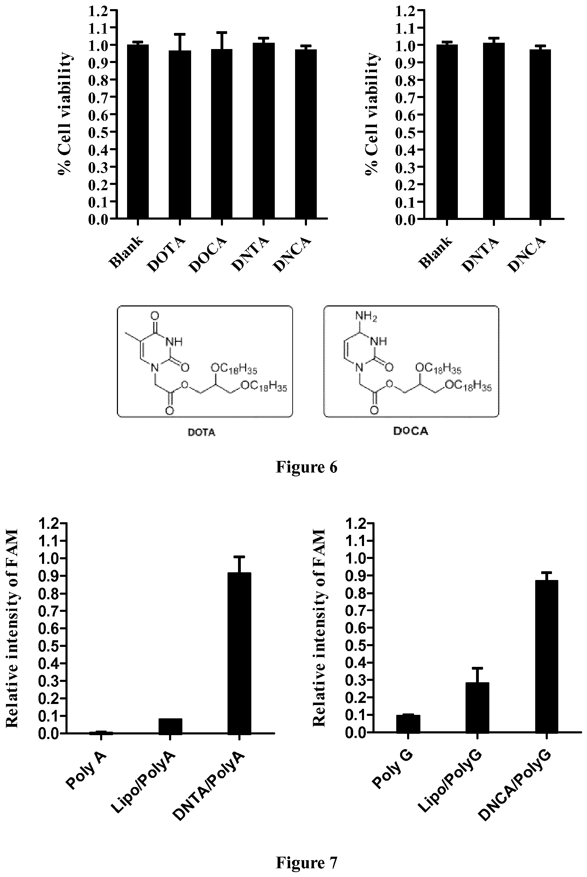

When base acetamide glycerin ether molecules should is to be used as novel biomaterials in the field of gene therapy, they must have good biocompatibility. Two base acetamide glycerin ether compounds (DOCA and DOTA) were studied for cytotoxicity using CCK-8 kit. The results showed that the two compounds (DNTA, DNCA) had no obvious cytotoxicity. After 72 hours of addition of the base acetamide glycerin ether compound at a concentration of 60 µM, the cell survival rate was close to 100%, indicating good biocompatibility (Fig. 6).

### 2. Transfection of nucleic acids

Taking DNTA and DNCA as examples, FAM-labeled polyA/G was used as a template to study the nucleic acid transfection abilities of thymine base acetamide glycerin ethers. The specific coating and transfection process is as follows:
DNTA or DNCA liposomes were formulated as mixed solutions with FAM-polyA or FAM-polyG at a base ratios of 5/1. The mixturex were heated to 96°C, then gradually reduced to 4°C (annealing), and placed at 4°C for 2 days. The cells used in the transfection experiments were Hela cells.
(1) Plating: 300,000 cells were added to the six-well plate, and the volume per well was 1.8 mL;
(2) After 18-24 h, the samples were diluted in an appropriate amount of opti-MEM, blowing and suction with a pipette to mix well, 20 µL of the mixed solutions were added to each well to make a final concentrations of FAM-polyA or FAM-polyG of 100 nM. The DNA-free and DNA-only wells were used as two negative controls, and the transfection reagent Lipofectamine 2000-DNA complex (the method of addition of transfection reagent and DNA group according to the protocol) was added as a positive control;
(3) Washing three times with 500 µL PBS;
(4) After 4 hours, the liquids in the culture wells were aspirated, after adding 250 µL of 5% trypsin each, they were placed in 1.5 mL EP tubes, the cells were fixed with 4% paraformaldehyde for 15 min;
(5) Centrifuging, discarding the supernatant, adding 400 µL PBS in each tube;
(6) Fluorescence intensity was measured by using flow cytometry;

Figure 7 shows the transfection results of DNTA- and DNCA liposomes against FAM-polyA/G. The experimental results show that DNTA- as well as DNCA liposomes can successfully transport FAM-polyA into cells.

### 3. Experiment for antisense nucleic acid uptake

The procedure was the same as the transfection experiments of DNCA- and DNTA-packed PolyG/PolyA (using DOCA and DOTA as controls), the cell line was selected from MCF-7 cell line.

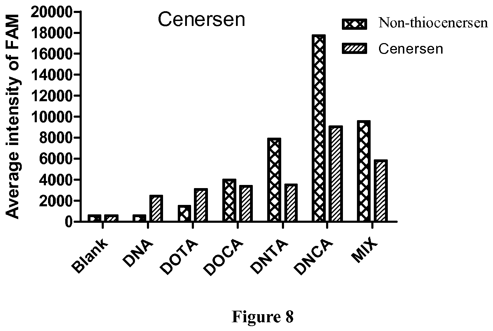

Figure 8 shows the effects of DNTA- and DNCA liposomes on the uptake of antisense nucleic acid Cenersen by MCF-7 cells. The experimental results showed that DNTA- and DNCA liposome could significantly increase the uptake ability of MCF-7 cells to antisense nucleic acid Cenersen compared with the blank group and DOCA/DOTA liposome groups.

## Claims

1. A base acetamide glycerin ether molecule which is **characterized by** an amphiphilic structure represented by formula (I), that is, with a base-1-acetic acid as polar head and a 2, 3-dialkoxyl-1-propylamine as non-polar tail, the two parts being linked by an amide bond to form the amphiphilic structure possessing both hydrophilicity and lipophilicity: 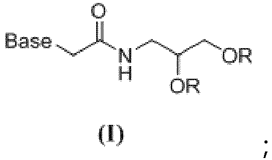 wherein the base contained therein, that is, the base group in Formula (I), is a common natural purine or pyrimidine base, that is, adenine, guanine, hypoxanthine, cytosine, thymine and uracil;
wherein the long chain aliphatic contained therein, that is, the R group in formula (I), is a saturated or unsaturated aliphatic carbon chain having between 8 and 25 carbons in length.

2. A chemical synthesis method for preparing a base-acetamide glycerin ether molecule according to claim 1, wherein the base-1-acetic acid activated derivative represented by formula (II) is reacted with 2, 3-dialkoxyl-1-propylamine represented by (III), to obtain a base acetamide glycerin ether molecule represented by formula (I) of claim 1; 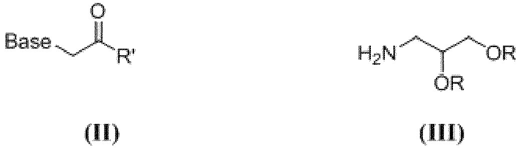 wherein a base-1-acetic acid activated derivative represented by formula (II) is used, wherein the R' group is chlorine or N-oxy-succinimide; to react with 2,3-dialkoxy-1-propylamine represented by formula (III), wherein the R group is a saturated or unsaturated aliphatic carbon chain with a length of between 8 and 25 carbons.

3. The chemical synthesis method according to claim 2, which comprises the steps of: (i) commercially purchasing, or, preparing from simple starting materials, the activated derivative of base-1-acetic acid represented by formula (II), and 2,3-dialkoxy-1-propylamine represented by formula (III); (ii) the base-1-acetic acid-activated derivative represented by formula (II), is reacted with the 2,3-dialkoxy-1-propylamine represented by formula (III) in an organic solvent, or reacted with addition of a base or ester condensation catalyst to obtain a target product represented by formula (I).

4. The chemical synthesis method according to claim 3, wherein the organic solvent used in the step (ii) is selected from the group consisting of acetonitrile, *N*,*N*-dimethylformamide, *N*-methylpyrrolidine, dichloromethane, and dichloroethane, a mixture of one or several of tetrahydrofuran, benzene or other aprotic solvents.

5. The chemical synthesis method according to claim 3 or 4, wherein one or several of 4-dimethylaminopyridine, triethylamine, pyridine, potassium hydroxide or sodium hydroxide are added in the step (ii) as additives, preferably, 4-dimethylaminopyridine is added as a catalyst, and pyridine or triethylamine is added as an organic base.

6. The use of base acetamide glycerin ether molecule according to claim 1 for the preparation of a substance having a supramolecular structure, preferably, the supramolecular structure is a liposome.

7. The base acetamide glycerin ether molecule according to claim 1 for use in the preparation of a transfection reagent for gene therapy.

## Patentansprüche

1. Basen-Acetamid-Glycerinether-Molekül, **gekennzeichnet durch** eine amphiphile Struktur, dargestellt durch Formel (I), das heißt mit einer Basen-1-Essigsäure als polarem Kopf und einem 2,3-Dialkoxy-1 -Propylamin als unpolarem Schwanz, wobei die zwei Teile durch eine Amidbindung verbunden sind, um die amphiphile Struktur zu bilden, die sowohl hydrophil als auch lipophil ist: 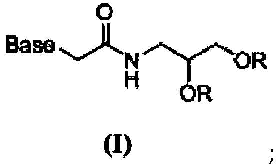 wobei die darin enthaltene Base, das heißt die Basengruppe in Formel (I), eine herkömmliche natürliche Purin-oder Pyrimidinbase ist, das heißt Guanin, Hypoxanthin, Cytosin, Thymin und Uracil;
wobei die darin enthaltene langkettige aliphatische Gruppe, das heißt die R-Gruppe in Formel (I), eine gesättigte oder ungesättigte aliphatische Kohlenstoffkette mit 8 bis 25 Kohlenstoffatomen ist.

2. Chemisches Syntheseverfahren zur Herstellung des Basen-Acetamid-Glycerinether-Moleküls nach Anspruch 1, wobei das durch die Formel (II) dargestellte Basen-1-Essigsäure-aktivierte Derivat mit 2,3-Dialkoxy-1-Propylamin, dargestellt durch (III), reagiert, um ein Basen-Acetamid-Glycerinether-Molekül zu erhalten, das durch die Formel (I) gemäß Anspruch 1 dargestellt ist; 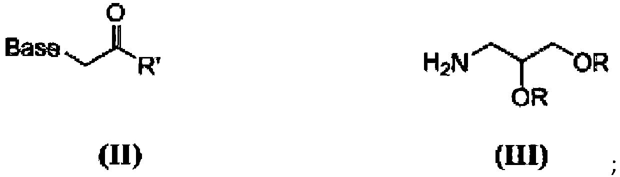 wobei ein durch Formel (II) dargestelltes Basen-1-Essigsäure-aktiviertes Derivat verwendet wird, wobei die R'-Gruppe Chlor oder N-oxy-Succinimid ist; um mit 2,3-Dialkoxy-1-Propylamin, dargestellt durch Formel (III), zu reagieren, wobei die R-Gruppe eine gesättigte oder ungesättigte aliphatische Kohlenstoffkette mit einer Länge zwischen 8 und 25 Kohlenstoffen ist.

3. Chemisches Syntheseverfahren nach Anspruch 2, umfassend die folgenden Schritte: (i) käufliches Erwerben oder Herstellen aus einfachen Ausgangsmaterialien des aktivierten Derivats einer Basen-1-Essigsäure der Formel (II) und 2,3-Dialkoxy-1-Propylamin der Formel (III); (ii) Reagieren des durch Formel (II) dargestellten Basen-1-Essigsäureaktivierten Derivats mit dem durch Formel (III) dargestellten 2,3-Dialkoxy-1-Propylamin in einem organischen Lösungsmittel, oder Reagieren unter Zusatz eines Basen- oder Ester-Kondensationskatalysators, um das durch Formel (I) dargestellte Zielprodukt zu erhalten.

4. Chemisches Syntheseverfahren nach Anspruch 3, wobei das in Schritt (ii) verwendete organische Lösungsmittel ausgewählt ist aus der Gruppe bestehend aus Acetonitril, *N*,*N*-Dimethylformamid, N-Methylpyrrolidin, Dichlormethan und Dichlorethan, einem Gemisch aus einem oder mehreren von Tetrahydrofuran, Benzol oder anderen aprotischen Lösungsmitteln.

5. Chemisches Syntheseverfahren nach Anspruch 3 oder 4, wobei in Schritt (ii) ein oder mehrere von 4-Dimethylaminopyridin, Triethylamin, Pyridin, Kaliumhydroxid oder Natriumhydroxid als Zusatzstoffe hinzugefügt werden, vorzugsweise wird 4-Dimethylaminopyridin als Katalysator hinzugefügt und Pyridin oder Triethylamin als organische Base hinzugefügt.

6. Verwendung des Basen-Acetamid-Glycerinether-Moleküls nach Anspruch 1 zur Herstellung einer Substanz mit einer supramolekularer Struktur, vorzugsweise ist die supramolekulare Struktur ein Liposom.

7. Basen-Acetamid-Glycerinether-Molekül nach Anspruch 1 zur Verwendung bei der Herstellung eines Transfektionsreagenzes zur Gentherapie.

## Revendications

1. Molécule d'éther de glycérine d'acétamide de base qui est **caractérisée par** une structure amphiphile représentée par la formule (I), c'est-à-dire avec un acide base-1-acétique en tant que tête polaire et une 2,3-dialcoxyl-1-propylamine en tant que queue non polaire, les deux parties étant liées par une liaison amide pour former la structure amphiphile possédant à la fois une hydrophilie et une lipophilie : 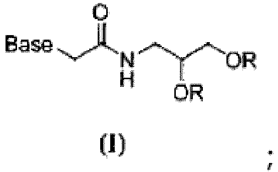 dans laquelle la base qu'elle contient, c'est-à-dire le groupe base dans la formule (I), est une base purine ou pyrimidine naturelle commune, c'est-à-dire une adénine, une guanine, une hypoxanthine, une cytosine, une thymine et un uracile,
dans laquelle l'aliphatique à longue chaîne qu'elle contient, c'est-à-dire le groupe R dans la formule (I), est une chaîne carbonée aliphatique saturée ou insaturée ayant une longueur entre 8 et 25 carbones.

2. Procédé de synthèse chimique pour préparer une molécule d'éther de glycérine d'acétamide de base selon la revendication 1, dans lequel le dérivé activé d'acide base-1-acétique représenté par la formule (II) est mis à réagir avec une 2,3-dialcoxy-1-propylamine représentée par (III), pour obtenir une molécule d'éther de glycérine d'acétamide de base représentée par la formule (I) selon la revendication 1, 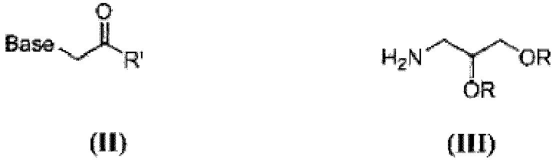 dans lequel un dérivé activé d'acide base-1-acétique représenté par la formule (II) est utilisé, dans lequel le groupe R' est un chlore ou un N-oxy-succinimide, pour réagir avec une 2,3-dialcoxy-1-propylamine représentée par la formule (III), dans lequel le groupe R est une chaîne carbonée aliphatique saturée ou insaturée d'une longueur entre 8 et 25 carbones.

3. Procédé de synthèse chimique selon la revendication 2, qui comprend les étapes consistant à : (i) acheter dans le commerce, ou préparer à partir de matières premières simples, le dérivé activé d'acide base-1-acétique représenté par la formule (II), et la 2,3-dialcoxy-1-propylamine représentée par la formule (III), (ii) le dérivé activé d'acide base-1-acétique représenté par la formule (II) est mis à réagir avec la 2,3-dialcoxy-1-propylamine représentée par la formule (III) dans un solvant organique, ou mis à réagir avec l'ajout d'un catalyseur de condensation de base ou d'ester pour obtenir un produit cible représenté par la formule (I).

4. Procédé de synthèse chimique selon la revendication 3, dans lequel le solvant organique utilisé dans l'étape (ii) est sélectionné dans le groupe consistant en l'acétonitrile, le *N*,*N*-diméthylformamide, la *N*-méthyl-pyrrolidine, le dichlorométhane et le dichloroéthane, un mélange d'un ou de plusieurs parmi le tétrahydrofurane, le benzène ou d'autres solvants aprotiques.

5. Procédé de synthèse chimique selon la revendication 3 ou 4, dans lequel un ou plusieurs parmi la 4-diméthylaminopyridine, la triéthylamine, la pyridine, l'hydroxyde de potassium ou l'hydroxyde de sodium sont ajoutés dans l'étape (ii) en tant qu'additifs, de préférence, la 4-diméthylaminopyridine est ajoutée en tant que catalyseur, et de la pyridine ou de la triéthylamine est ajoutée en tant que base organique.

6. Utilisation d'une molécule d'éther de glycérine d'acétamide de base selon la revendication 1 pour la préparation d'une substance ayant une structure supramoléculaire, de préférence, la structure supramoléculaire est un liposome.

7. Molécule d'éther de glycérine d'acétamide de base selon la revendication 1 pour une utilisation dans la préparation d'un réactif de transfection pour la thérapie génique.
